# EUROPEAN PATENT SPECIFICATION

(11) **EP 3 629 909 B1**
(45) Date of publication and mention of the grant of the patent: **26.11.2025**
(21) Application number: 18805101.5
(22) Date of filing: 23.05.2018
(51) Int. Cl.: A61B 5/0225, A61B 5/022, A61B 5/00

(54) **METHOD FOR CORRECTING CUFF PRESSURE IN A NON-INVASIVE BLOOD PRESSURE MEASUREMENT**
VERFAHREN ZUR KORREKTUR DES MANSCHETTENDRUCKS BEI DER NICHTINVASIVEN BLUTDRUCKMESSUNG
MÉTHODE DE CORRECTION DE LA PRESSION DE BRASSARD DANS UNE MESURE DE PRESSION SANGUINE NON INVASIVE

(30) Priority: 23.05.2017 US 201762509877 P; 21.05.2018 US 201815984634
(43) Date of publication of application: 08.04.2020
(73) Proprietor: Becton, Dickinson And Company, Franklin Lakes, New Jersey 07417 (US)
(72) Inventor: SETTELS, Jacobus Jozef, Gerardus Maria, Irvine CA 92614 (US)
(74) Representative: Eisenführ Speiser
(86) International application number: PCT/US2018/034078
(87) International publication number: WO 2018/217858

(56) References cited:
- WO-A1-96/03914
- US-A- 4 475 554
- US-A1- 2002 099 298
- US-A1- 2011 105 918
- US-A1- 2013 023 777
- US-A1- 2015 201 852
- US-A1- 2016 100 805
- US-B2- 6 632 181

## Description

The present invention relates to a method for correcting cuff pressure in a non-invasive continuous pressure blood pressure measurement, as well as to a device for carrying out said method.

It has been known for several years to measure blood pressures wherein a pressure cuff is placed around a body extremity, such as a finger. EP 0 048 060 for instance describes that the pressure of a fluid inside the pressure cuff is controlled on the basis on a signal of an optical plethysmograph by a pressure valve, in turn controlled by a control loop.

US 2015/0201852 A1 relates to a method and a device for continuously determining blood pressure in a non-invasive manner using a photoplethysmographic system which has at least one light source and at least one light detector that is arranged with a mount on a body part containing an artery. According to this document, a device is provided with which the contact pressure of the mount on the body part can be adjusted and/or changed depending on the mean blood pressure. US4475554 discloses a method and device utilizing a flexible finger cuff with a photoplethysmograph and a dynamic compressor to continuously track arterial pressure by maintaining a constant arterial volume through real-time cuff pressure adjustments. Additionally, US6632181B2 describes a technique for rapidly determining blood pressure within a single cardiac cycle by applying an external pressure to generate a range of transmural pressures and using simultaneous tonometer and plethysmograph measurements to identify a compliance peak.

The signal of the optical plethysmograph is representing the volume of blood inside the blood vessels of the finger. The more blood inside the vessels, the more light from a light source of the plethysmograph is absorbed, which results in a lower signal of the detector side of the plethysmograph (and vice versa). During every heartbeat, blood is forced through the blood vessels in the finger, causing the vessels to expand and allow more blood to flow through the vessels. This also causes a volume increase of the vessels, and thus a signal decrease of the plethysmograph.

In the known method, the cuff pressure of the pressure cuff is controlled such that the signal of the plethysmograph, and thus the volume of blood inside the blood vessels, is kept constant. The pressure exerted on the internal blood vessel walls is continuously counteracted by a pressure exerted by the pressure cuff on the external blood vessel walls, which results in a constant diameter of the blood vessels and an unloading of the vessels. The counter pressure exerted by the pressure cuff is a measure for the actual blood pressure inside the blood vessel, and allows for a continuous non-invasive blood pressure measurement.

This control is arranged such that at any moment the difference between a servo reference level or set point value for the diameter of the blood vessels and the actual plethysmograph signal or real value is minimized, ideally to zero. The servo-reference level in the known method is initially determined automatically and the servo feedback control is operated in a way such that the cuff pressure continuously corresponds substantially with the momentary arterial pressure under the cuff both for pulsations and for absolute pressure level.

The known method requires correction of the reference or set point value over time. This correction is required mainly due to changes in the physiological status of the measured body part. US 4,510,940 for instance describes a method and a device for correcting the cuff pressure in the indirect, non-invasive and continuous measurement of the blood pressure in a part of the body by using a plethysmograph in a fluid-filled pressure cuff, an electronic control circuit, and an electric pressure valve. The cuff pressure is controlled by the plethysmograph signal in closed-loop operation with the aid of a servo-reference level obtained via a memory circuit. The servo-reference level, in operation of the device, is adjusted by opening the closed loop of the control circuit for a short interval, after which, in open-loop operation the cuff pressure is adjusted at an intermediate pressure derived from the pressure last measured and the servo-reference level is adjusted via the memory circuit.

Although these methods work, the servo-reference level is adjusted or corrected based only on volume measurements of the plethysmograph as a function of time, over only a part of the heart beat duration, and with only constant cuff pressures. These reference computations make assumptions on the blood pressure levels inside the vessels over the time of the correction computation and are sensitive to external influences. Also, these methods are relatively slow. For adjustment determination of the reference level, often several heartbeats are required to determine the response in volume to many different pressures in the pressure cuff, during which heartbeats a measurement of the blood pressure cannot be performed.

It is therefore an objective of the present invention to provide an improved method for correcting cuff pressure in a non-invasive continuous blood pressure measurement.

The invention thereto provides a method for correcting cuff pressure in a non-invasive blood pressure measurement with a plethysmograph in a fluid filled pressure cuff wrapped around a body part, such as a finger, comprising the steps of: varying pressures inside the pressure cuff and measuring the corresponding signals of the plethysmograph; determining a value of the plethysmograph signal corresponding to a predetermined arterial volume, in particular the un-stretched or unloaded arterial volume; and setting the determined value as set point; comparing measured plethysmograph signals with the set point for a servo system; adjusting cuff pressure in the pressure cuff to minimise the difference between the measured signals and the set point; monitoring the cuff pressure for a certain amount of heartbeats; storing the pressure adjustments of the cuff during at least one first heartbeat; applying a varying cuff pressure to the pressure cuff during at least one second heartbeat and measuring the corresponding signals of the plethysmograph; using the stored pressure adjustments of the at least one first heartbeat, and the cuff pressure and plethysmograph signal of the at least one second heartbeat, to determine a pressure-volume relation of the blood vessel under the cuff; and determining a value of the plethysmograph signal corresponding to predetermined arterial volume, preferably an un-stretched arterial volume, based on the determined relation; and setting the determined value as new set point. A fluid according to the invention may for instance be a liquid such as water, or a gas like air.

In the method according to the invention, the blood pressure inside the blood vessel is followed for a certain period of time, to monitor the blood pressure. To do so, a first set point is determined, which approximately corresponds to an unloaded volume of the blood vessel. The pressure in the cuff is adjusted to keep the volume constant, and this pressure is thus a measure for the internal blood pressure (or intra-arterial pressure). Due to multiple factors, this set points typically drifts with time such that calibration, or setting a new set point, is required after a certain period of time.

The set point, or unloaded volume of the blood vessel, can be determined based on a pressure-volume relation between the transmural pressure, which is the internal blood pressure (or intra-arterial pressure) minus the cuff pressure, and the volume of blood inside the blood vessel. The volume is determined by the plethysmograph signal. In the determination of the transmural pressure, the cuff pressure is available and adjustable, but the present (or actual) internal blood pressure is an unknown.

However, changes between blood pressure waveforms generally occur gradually, and if they differ from one heartbeat to the next, the changes are mostly in pulse pressure, which changes can be predicted based on changes of the duration of the heart beat and compensated for. In the determination of the pressure-volume relation according to the invention, the second set of blood pressure waveform for the second heart beat is assumed to be identical to the first set of blood pressure waveform or assumed to be correctly adapted from the first based on heart beat interval. Instead of the present blood pressure in the blood vessel, the blood pressure of the first, preceding set is used. The pressure of the cuff can now be chosen dynamically to cover a range of pressures suitable for the determination of the pressure-volume relation.

The advantage of such method is that a large dynamic range of transmural pressures may be used to compute an actual pressure-volume relation of the blood vessel on which the set point is based, whereas in the prior art the set point is based only a determination on volume information over time.

Another advantage of such method is that it is relatively fast. Because the internal blood pressure, or intra-arterial pressure, is assumed to be known, only one or two heartbeats are required which can be used to dynamically cover a large range of transmural pressures, as the cuff pressure may be varied based on the information from the assumed intra-arterial pressure.

Another advantage of such method is that it allows the analysis of visco-elastic properties of the arterial wall of the blood vessel by determining the phase relationship, compliance and hysteresis characteristics between the transmural pressure dynamics and the resulting volume dynamics.

The beat duration of the second heart beat can be determined from the first heartbeat, and the pulse pressure of the second heart beat can be adapted when the beat duration differs from the first heartbeat.

The at least one first heartbeat and the at least one second heartbeat may comprise the same number of heartbeats, for instance one or two heartbeats.

The method according to the invention may repeat the monitoring of blood pressure and correcting the set point after a number of heartbeats. Because the correction of the set point only takes one or a few heartbeats to perform, and the blood pressures during these heartbeat(s) are available as predicted by simulation, only a little bit of actual blood pressure measurement is lost. This allows for as many corrections over time as needed, which improves the quality of the blood pressure determination.

The varying cuff pressure applied during the at least one second heartbeat may comprise a pressure below venous pressure in the body part under the cuff and distal to it. In the finger, the use of a pressure cuff to determine arterial blood pressure in the described way typically blocks or impedes to a major extent the venous return of blood, since the cuff pressure, equalling the arterial pressure, exceeds the venous pressures. The venous blood vessels under the cuff are thus typically collapsed during blood pressure measurements with pressure cuffs. Same is true for the capillary bed under the cuff. This will, after a period of time, result in venous congestion in the body part distal to the cuff, increased venous and capillary pressure and potentially reduced oxygenation in the extremity of the body part, such as the fingertip. When the varying cuff pressure applied during the at least one second heartbeat comprises a pressure below the venous pressure in the fingertip, it allows venous return from the distal part of the extremity to the collecting veins, and avoids to abovementioned congestion problems. In order to achieve this, the cuff pressure below fingertip venous pressure may lie between 0 and diastolic arterial pressure, preferably between 0 and 50 mmHg, in particular around 30 mmHg.

The varying cuff pressure applied during the at least one second heartbeat may be a dynamic pressure waveform, such as a sinusoidal waveform. Because the arterial pressure is assumed to be known during the second heartbeat, the pressure-volume relation may be determined with any cuff pressure. The pressure-volume relation relies on the transmural pressure over the arterial wall and the corresponding volume information based on the plethysmograph signal. The transmural pressure is the difference between the cuff pressure and the arterial pressure. As the blood pressure has a somewhat sinusoidal progression itself also, a sinusoidal cuff pressure waveform in counter phase with the blood pressure waveform would allow to generate transmural pressures over a relative large range along with its volume information from the plethysmograph, both positive and negative transmural pressures.

The varying cuff pressure applied during the at least one second heartbeat may also be a ramp waveform, wherein preferably the lowest pressure below diastolic pressure in the body part allows venous return in the body part, and/or the highest pressure is above systolic pressure in the body part. The ramp pressure can be in phase or in counter phase with the blood pressure dynamics, or any phase relationship in between. A ramp in pressure is relatively easy to apply. The ramp waveform may comprise multiple ramps, such that the waveform is more like a saw tooth waveform.

The varying cuff pressure applied during the at least one second heartbeat may be chosen such that the transmural pressure between the blood vessel of the body part and the cuff pressure is set over a predetermined range. The transmural pressure depends on the arterial pressure and the cuff pressure, of which the arterial pressure is known, and the cuff pressure can be set. In order to determine the transmural pressure over a predetermined range, the cuff pressure can be varied based on the arterial pressure. The varying pressure profile may thus be determined based on the stored blood pressure of the at least one first heartbeat.

The method may further comprise the step of determining the central blood pressure based on the measured blood pressure inside the body part, preferably the finger. When the blood pressure inside the body part is transformed in the central blood pressure, measurements of different body parts may be compared, as all can be related to central blood pressure. Central blood pressure is for instance the aortic blood pressure or the brachial blood pressure.

The step of using the stored pressure adjustments of the at least one first heartbeat, and the cuff pressure and plethysmograph signal of the at least one second heartbeat, to determine a pressure-volume relation for instance involves plotting the transmural pressure against the volume signal of the plethysmograph. In each heartbeat, this pressure/volume relation substantially forms a loop, with an upswing and a downswing distinguished from each other because of the hysteresis of the arterial wall. The gradient, or angle/steepness of these swings is a measure for the compliance of the blood vessel being measured. In order to better study this gradient or steepness, instead of the volume one could take the first derivative of the volume signal, and plot this derivative over the transmural pressure. The location where this first derivative is maximal (or where a second derivative would be zero) represents the transmural pressure with maximal compliance. This transmural pressure could be used in the pressure-volume relation to determine the volume where compliance is maximal, and it is this volume which could serve as new set point according to the invention.

The invention further relates to a device for correcting cuff pressure in a non-invasive blood pressure measurement with a plethysmograph in a fluid filled pressure cuff wrapped around a body part, such as a finger, comprising a pressure cuff, provided with: a bladder, for wrapping around the body part and applying counter pressure; a light source, for sending light through the body part, and a light detector, for detecting the light passed through the body part and for providing a signal in dependence of the amount of detected light; a pressure generator, for supplying pressurized fluid to the bladder; a controller, for adjusting the pressure of the fluid supplied to the bladder based on the signal, and for determining the blood pressure inside the body part; a memory, for storing the determined blood pressure of at least one first heartbeat; wherein the controller is arranged to apply a varying dynamic cuff pressure during at least one second heartbeat, and to receive the signal during the at least one second heartbeat, and wherein the controller is arranged to determine a pressure volume relation based on the stored blood pressure of the at least one first heartbeat, the signal of the at least one second heartbeat and the varying cuff pressure applied during the at least one second heartbeat.

The device may further comprise a pressure sensor arranged to determine the pressure inside the bladder. The pressure inside the bladder is an indication of the blood pressure inside the body part when the pressure is adjusted to maintain a constant plethysmograph signal.

The invention will be explained by means of the non-limiting working examples depicted in the following figures. Specifically:
- figure 1 schematically shows a device for non-invasive blood pressure measurements according to the present invention;
- figure 2 schematically shows a first approximate determination of the set point according to the present invention;
- figure 3 schematically shows the monitoring of blood pressure after the first setting of the set point according to the present invention;
- figures 4A-4E schematically shows the determination of a new, changed, set point according to the present invention; and
- figure 5A-5E shows the actual determination of a new, changed, set point according to the present invention using a ramp as the varying cuff pressure and
- figure 6A-6E shows the actual determination of a new, changed, set point according to the present invention using a sinusoidal waveform as the varying cuff pressure.

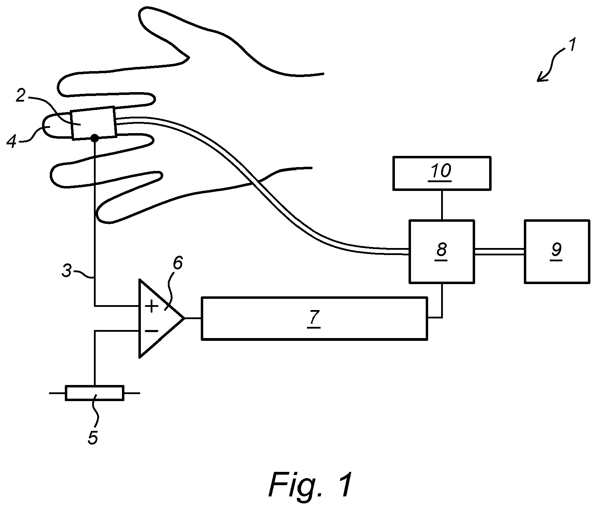

Figure 1 schematically shows a device (1) for non-invasive blood pressure measurements, comprising a pressure cuff (2), which generates a signal (3) based on the detected light. This signal (3), representative for the volume of blood in the finger arteries (4) is compared to a set-point (5) by a comparator (6), which comparison is then communicated to a controller (7). Based on the information, the controller (7) in turn controls a control valve (8). The valve (8) regulates the pressure supplied to the pressure cuff (2) by a pump (9). The pressure supplied to the pressure cuff (2) is measured by a transducer (10).

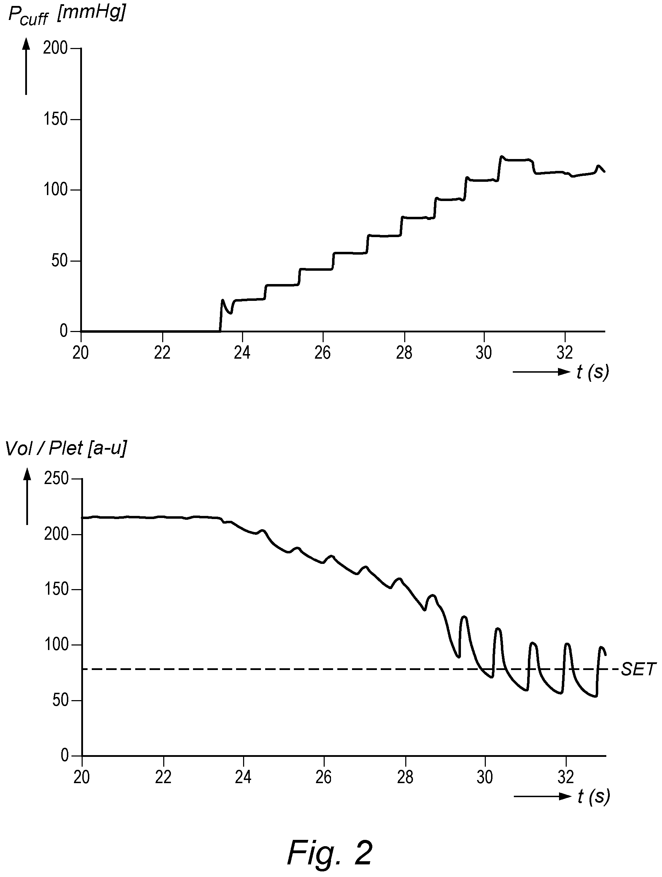

Figure 2 schematically shows a first determination of the first set point (SET). In the upper graph, the pressures (P_{cuff}) inside the pressure cuff are plotted over time (t). The pressures are varied in this example by applying them in a step pattern with increasing cuff pressures. Such pattern can however be selected from a number of patterns, also for instance including a ramp pattern.

In the bottom graph, the corresponding plethysmograph signal is shown. At low cuff pressures (on the left of the graph), the arteries of the finger are open and a large amount of blood is present in the arteries. The plethysmograph signal (PLET), corresponding to the amount of blood in the measured arteries, is thus relatively high. At high cuff pressures (on the middle/right of the graph), the cuff exerts a large pressure on the finger and the arteries, causing them to collapse at least partially during the diastolic phase of the heartbeat. This collapse of the arteries reduces the blood volume, and thus results in a decreased plethysmograph signal.

The blood pressure in the arteries also pulsates with every heartbeat. Every heartbeat blood is expelled from the heart into the aorta during systole, which is followed by the filling of the heart during diastole. The pressure in the arteries follows the same pattern, and increases due to the expulsion of blood, and decreases during filling of the heart. This pulsation causes an expansion of the arteries in the finger, and thus in a varying volume of blood in the arteries. This varying volume also results in a varying plethysmograph signal.

The variations in the plethysmograph signal are small when the cuff pressure is high (for instance above systolic pressure), as the arteries are subjected to high external pressures, working against the internal pressure variations. The variations in the plethysmograph signal are also small when the cuff pressure is low (for instance below diastolic pressure), as then arteries are loaded by the blood pressure from within.

When the external pressure of the pressure cuff and the internal blood pressure of the arteries are close or equal, they balance each other out. This is also referred to as the unloaded state of the arteries, wherein the pressure difference over the arterial walls (also referred to as transmural pressure) is zero. In this state, the varying blood pressure causes, relatively unobstructed and directly, the expansion of the arteries. It is in this state that the variations in the plethysmograph signal are largest. The initial set point, is for instance chosen as the middle between the top and bottom of the plethysmograph signal where the variations are largest. This procedure does not provide a very accurate volume set point, but it suffices to start a measurement with the new set point adjustments as described, which quickly converges to a stable and more accurate volume set point value.

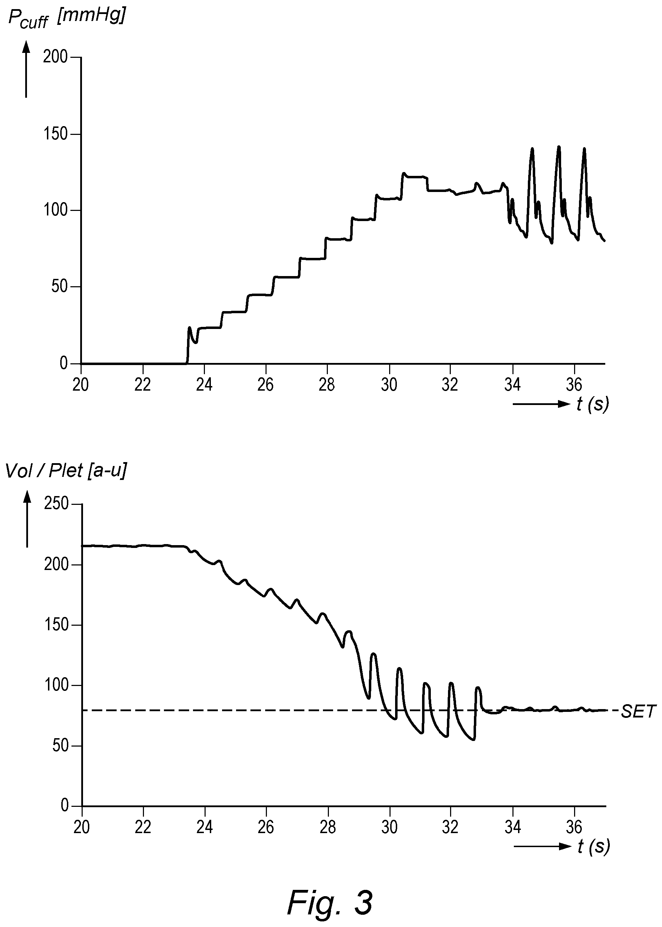

Figure 3 schematically shows the monitoring of blood pressure after the setting of the initial set point. The pressure in the cuff (P_{cuff}) is varied such that the plethysmograph signal (PLET) is kept constant, at the set point value. The pressure in the cuff is now representing the blood pressure inside the arteries.

The set point, representing the predetermined or unloaded volume of the arteries, changes over time. Performing the same step pattern in cuff pressures to determine the changed set point takes a relative long time to do, and is not very accurate and the blood pressure measurement is then temporarily not available.

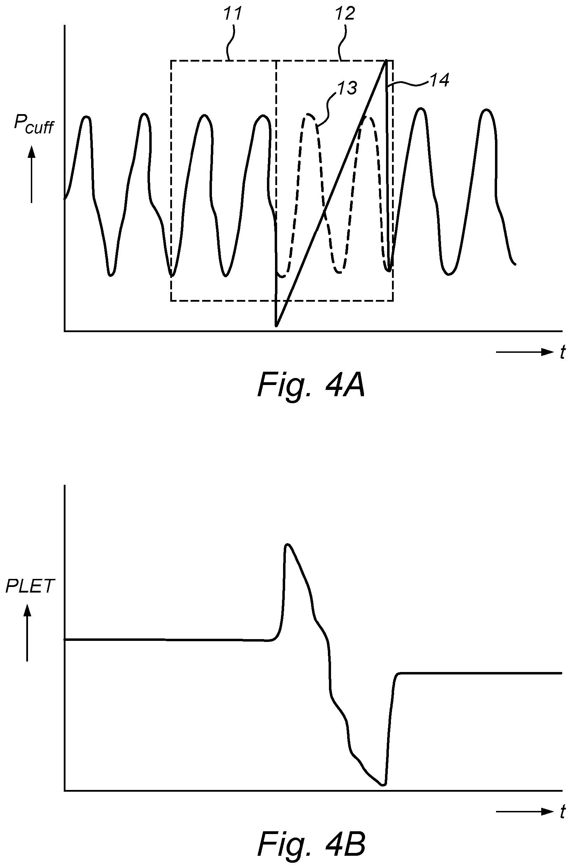

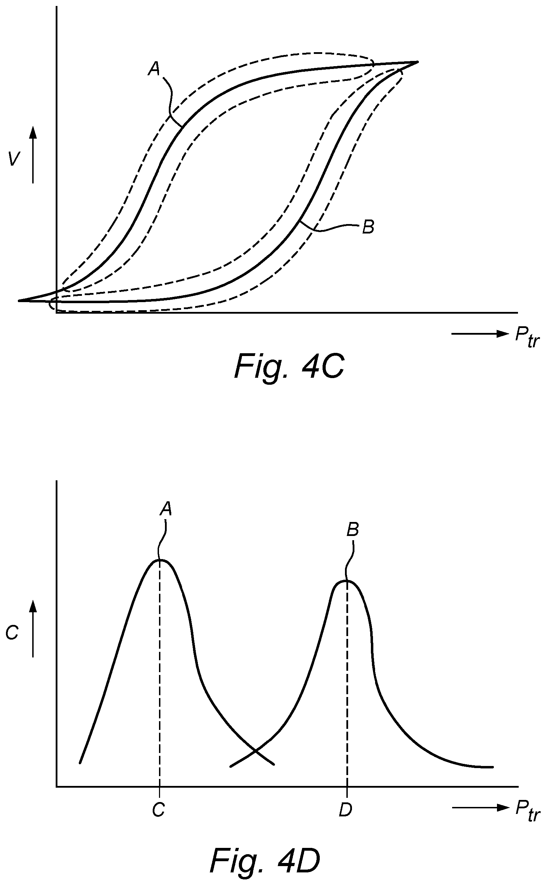

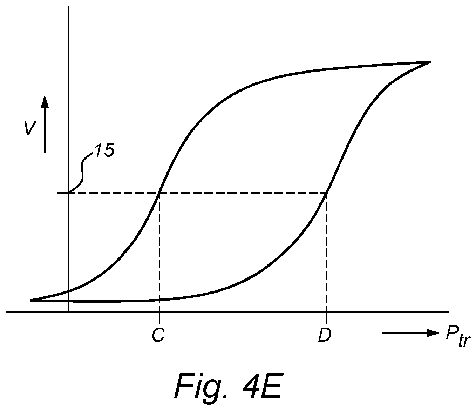

Figure 4 schematically shows the determination of the information leading to a new, changed, set point. Figure 4A shows a changing pressure in the cuff, P_{cuff}, and figure 4B shows the changing plethysmograph signal over that time, PLET. In figure 4A, the blood pressure is monitored for a number, for example four, heartbeats before determination of the new set points. During this monitoring the plethysmograph signal (PLET) in figure 4B is kept constant at or around the previously determined set point, and the pressure in the cuff is adjusted accordingly.

The cuff pressures, representing blood pressures, of the last two heartbeats (11) are stored. In figure 4 two heartbeats are stored, but the same can hold for one or more heartbeats. For the next two heartbeats (12), the blood pressures are assumed to be identical to the stored heartbeats (11), as indicated with the dotted line (13) in the upper graph of figure 3. During these two heartbeats (12), a varying cuff pressure pattern (P_{cuff}) is applied to the pressure cuff, as indicated by e.g. the ramp (14). The plethysmograph signal (PLET) is measured over this range of cuff pressures.

The blood pressures during the two heartbeats are known, as they are assumed to be the same as the previous two heartbeats. In addition, they can be slightly modified based on the variation in heart beat interval. The cuff pressures (P_{cuff}) during the two heartbeats are the pressures according to the ramp (14), and the blood volume in the arteries is provided by the plethysmograph signal (PLET). The pressure over the arterial walls, or transmural pressure, is the difference between the cuff pressure and the arterial blood pressure, and can thus be determined. The volume can subsequently be plotted as a function of transmural pressure.

Figure 4C schematically shows a pressure volume relation according to the invention, which can be obtained using the steps described with regard to figures 4A and 4B. For sake of simplicity, the relation is shown for a single heartbeat. On the horizontal axis, the transmural pressure (Ptr) is shown, which pressure is the internal blood pressure, or arterial pressure, minus the cuff pressure. On the vertical axis, a volume (in internal units) is shown, which is provided by the signal received by the plethysmograph. In the shown relation, two curves (A, B) can be identified, curve A being related to the inflow and curve B being related to the outflow of blood. The gradient, or steepness, of each of the curves is a measure for the compliance of the blood vessel, and is the derivative of these curves.

Figure 4D schematically shows the (first) derivative of these curves (A, B) schematically. For each of these curves (A, B) a maximum can be determined, which corresponds to a certain transmural pressure (Ptr), one for curve A (C), and one for curve B (D). Figure 4E schematically shows how these transmural pressures where compliance is maximal can be used to determine a new set point. The new (volume) set point is the volume (15) in the pressure-volume relation as for instance shown in figure 4C which corresponds to the transmural pressures where compliance is maximal. This volume (15), will be the new (volume) set point (Vₛₑₜ) of the plethysmograph. It should be noted that the actual transmural pressure is used in this method (just) for referencing, and its precise value is of minor relevance. When the two curves (A, B) would have volumes (15) which are not coinciding, but would be different, the new set point could for instance be the average volume of the two volumes (15).

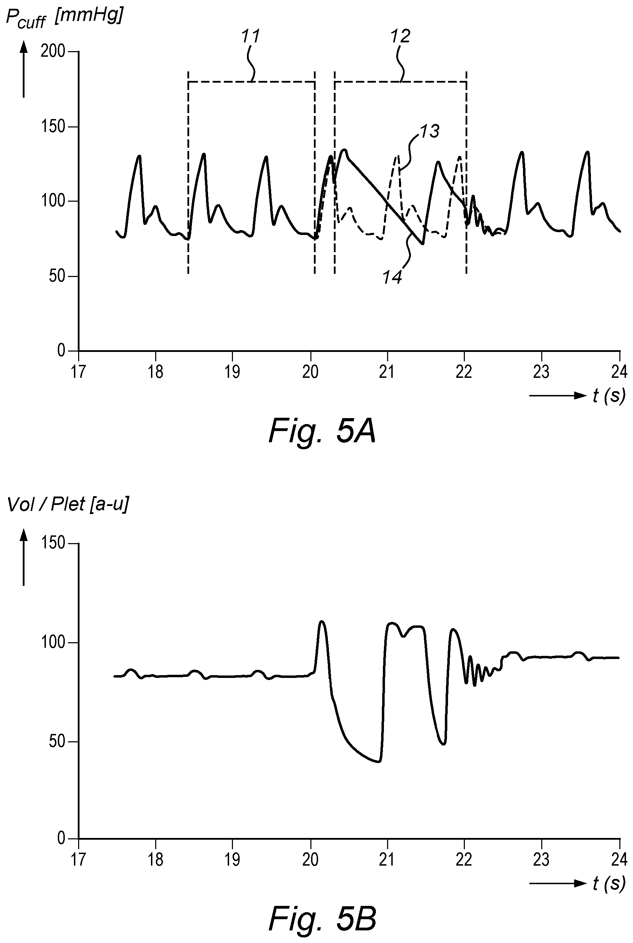

Figures 5A-5E show the determination of a new, changed, set point according to figure 4 in an actual measurement, using a ramp as the varying cuff pressure. The steps of the determination are according to the ones of figure 4.

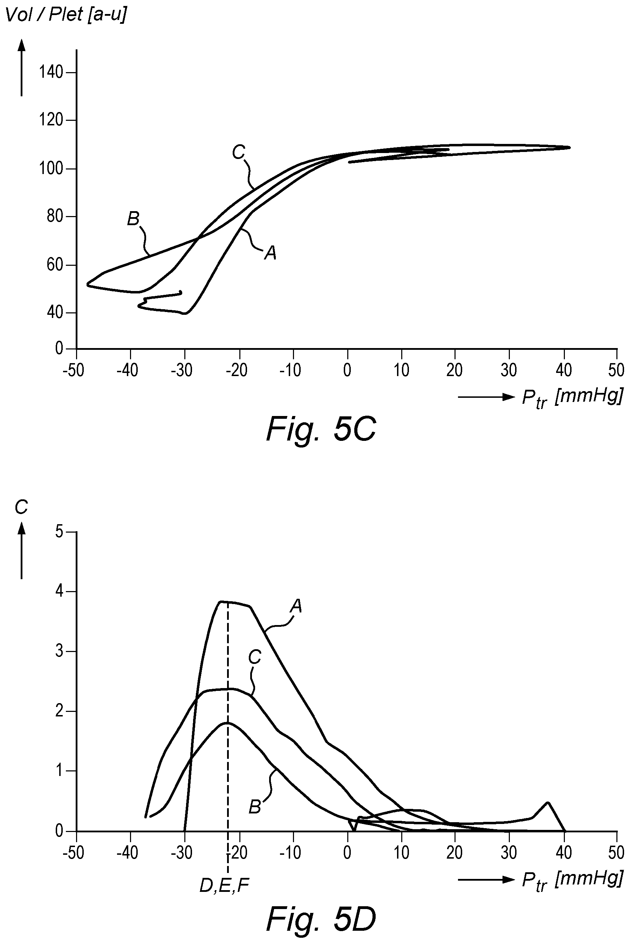

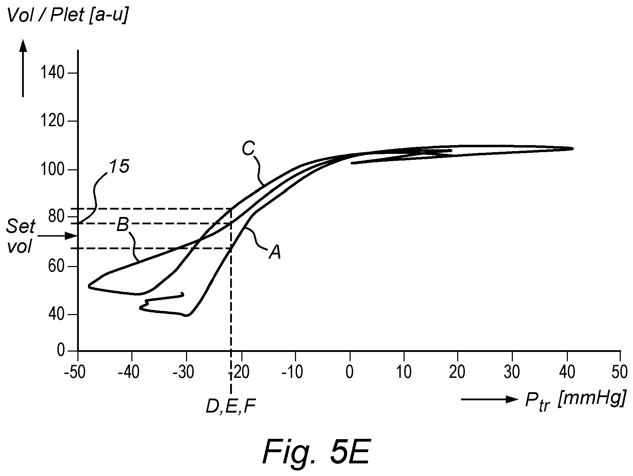

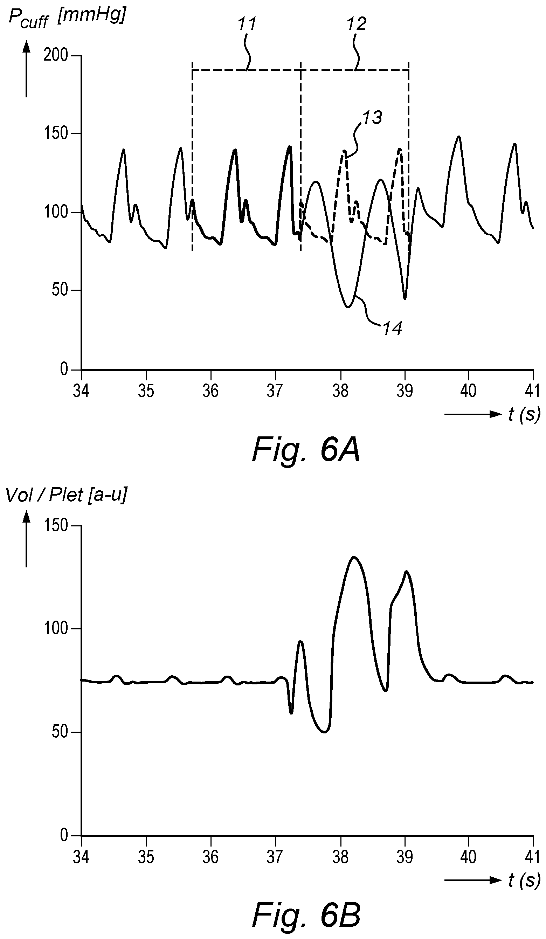

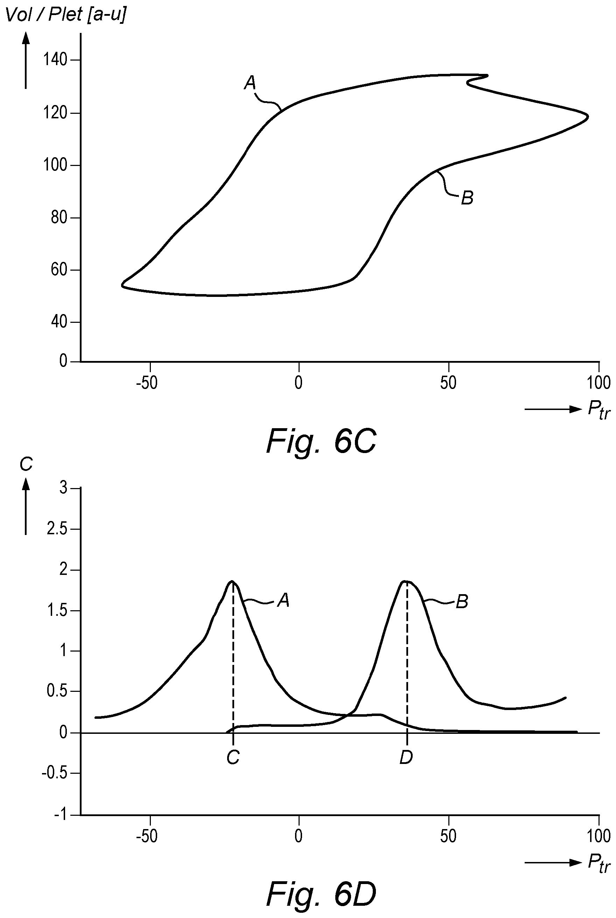

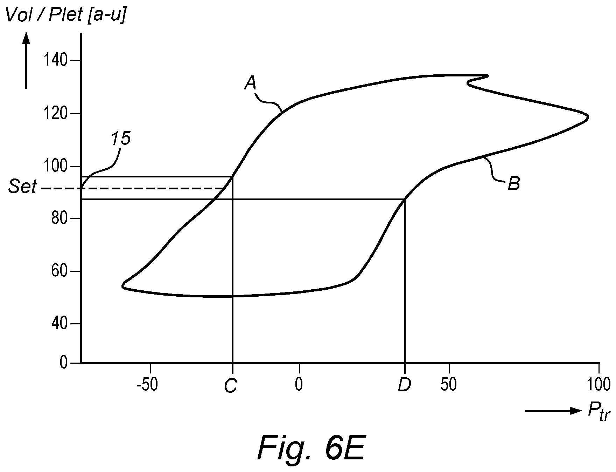

Figures 6A-6E show the determination of a new, changed, set point in an actual measurement, using a sinusoidal waveform as the varying cuff pressure. The steps of the determination are according to the ones of figure 4 and 5.

By including cuff pressure profiles that contain both transitions from low pressure, e.g. below diastolic to high pressure, e.g. above systolic and transitions from high to low pressure, the phase relationship between pressure and volume will show in the pressure-volume diagram as hysteresis if that is present, and provide information on the arteries. The hysteresis will provide at least two different values for the unloaded volume at zero transmural pressure. These values can be used to compute a more accurate determination of the set point at the unloaded volume, by computing an average of the two or more values, where the average can be a weighted average.

Because of its visco-elastic properties, the arterial wall of the body part under the cuffs has low pass filtering characteristics which can be determined by applying a step function in transmural pressure up and down around a predetermined cuff pressure level; this level is preferably at mean arterial pressure or at mid pressure, half way in between systolic and diastolic pressure. The varying cuff pressure in this case has the profile of a step up or down, followed by cuff pressure tracking the pressure inside the finger artery over a certain period, thus keeping the transmural pressure constant over a certain period which preferably is in the order of several 100 milliseconds. The resulting volume transient will have a characteristic which can be approximated by a first order time constant and estimated by the controller. This time constant will vary from person to person, and will vary over time within a person. For better robustness, the average value of the time constant up and the time constant down can be determined. The resulting time constant value can be used to individualize an important compensation component in the servo feedback loop that maintains the volume to the set point value; to individualize to each patient, and to adapt to changes over time. As a result, the servo will have improved bandwidth which will result in higher quality waveform details. The time constant can also be determined using a step in cuff pressure up or down, followed by a constant cuff pressure over a certain period. In this case, however, the transmural pressure will not be kept constant and the resulting time constant estimate will have a bias depending on the individual pressure shape in the finger artery.

## Claims

1. A method for correcting cuff pressure in a non-invasive continuous blood pressure measurement with a plethysmograph in a fluid filled pressure cuff (2) wrapped around a body part, the method comprising the steps of:
a) varying pressures (P_{cuff}) inside the pressure cuff (2) and measuring the corresponding signals of the plethysmograph;
b) determining a value of the plethysmograph signal (PLET) corresponding to an unloaded arterial volume; and setting the determined value as an initial set point (SET);
c) comparing measured plethysmograph signals (PLET) with the set point;
d) adjusting cuff pressure (P_{cuff}) in the pressure cuff (2) to minimise the difference between the measured signals (PLET) and the set point (SET);
e) monitoring the cuff pressure for a number of heartbeats;
**characterized by** the method further comprising the steps of:
f) storing the pressure adjustments of the cuff during at least one first heartbeat (11);
g) applying a varying cuff pressure (P_{cuff}) to the pressure cuff (2) during at least one second heartbeat (12) and measuring the corresponding signals (PLET) of the plethysmograph;
h) using the stored pressure adjustments of the at least one first heartbeat (11), and the cuff pressure (P_{cuff}) and plethysmograph signal (PLET) of the at least one second heartbeat (12), to determine a pressure-volume relation;
i) determining a value of the plethysmograph signal (PLET) corresponding to an unstretched arterial volume, based on the determined relation; and setting the determined value as a new set point (SET).

2. The method according to claim 1, comprising step j) of repeating steps c) to i).

3. The method according to claim 1, wherein the varying cuff pressure (P_{cuff}) applied in step g) comprises a pressure below venous pressure in the body part distal to the cuff (2).

4. The method according to claim 3, wherein the pressure below venous pressure in the body part distal to the cuff (2) reaches values below diastolic blood pressure, preferably between 0 and 50 mmHg, in particular around 30 mmHg.

5. The method according to claim 1, wherein the varying cuff pressure (P_{cuff}) applied in step g) is a dynamic pressure waveform, in which the lowest pressure is below diastolic pressure in the body part, which allows venous return in the body part, and the highest pressure is above systolic pressure in the body part.

6. The method according to claim 1, wherein the varying cuff pressure (P_{cuff}) applied in step g) is a ramp waveform in which the lowest pressure is below diastolic pressure in the body part, which allows venous return in the body part, and the highest pressure is above systolic pressure in the body part.

7. The method according to claim 1, wherein the varying cuff pressure (P_{cuff}) applied in step g) comprises a transition from a low pressure below diastolic pressure to a high pressure above systolic pressure, and thereafter multiple transitions between said low and high pressures.

8. The method according to claim 1, and further comprising a step of applying a cuff pressure (P_{cuff}) having a step function around a predetermined cuff pressure level, which level is one of (i) mean arterial pressure and (ii) a pressure halfway between systolic and diastolic pressure.

9. The method according to claim 1, wherein the pressure-volume relation is determined by the signal of the plethysmograph (PLET) and the transmural pressure (Ptr) between the blood vessel of the body part and the cuff pressure (P_{cuff}).

10. The method according to any of the preceding claims, wherein the varying cuff pressure (P_{cuff}) applied during step g) is chosen such that the transmural pressure (Ptr) between the blood vessel of the body part and the cuff pressure (P_{cuff}) is set over a predetermined range.

11. The method according to any of the preceding claims, wherein the varying cuff pressure (P_{cuff}) applied during step g) is determined based on stored blood pressure of the at least one first heartbeat (11).

12. The method according to any of the preceding claims, wherein in step b), the set point value is the volume wherein the amplitude of the plethysmograph signal is maximal.

13. The method according to any of the preceding claims, comprising the step of determining the central blood pressure based on the measured blood pressure inside the body part, preferably the finger.

14. The method according to any of the preceding claims, wherein in step i), the new set point is determined in relation to the volume where a compliance of the measured body part, such as the finger artery, is maximal.

15. Device (1) for correcting cuff pressure in a non-invasive blood pressure measurement with a plethysmograph in a fluid filled pressure cuff (2) wrapped around a body part, such as a finger, comprising:
a) a pressure cuff (2), provided with:
a. a bladder, for wrapping around the body part and for applying a counter pressure;
b. a light source, for sending light through the body part; and
c. a light detector, for detecting the light passed through the body part and for providing a signal (PLET) in dependence of the amount of detected light;
b) a pressure generator (9) configured to supply pressurized fluid to the bladder;
c) a controller (7) configured to adjust the pressure of the fluid supplied to the bladder based on the signal (PLET), to vary pressures (Pcuff) inside the pressure cuff (2), to measure the corresponding signals (PLET), to determine a value of the plethysmograph signal (PLET) corresponding to an unloaded arterial volume to set an initial set point (SET), to compare measured plethysmograph signals (PLET) with the set point (SET), to adjust the pressure to minimise the difference between the measured signals (PLET) and the set point (SET), to monitor the cuff pressure for a number of heartbeats, and to determine the blood pressure inside the body part; and
d) a memory,
**characterized in that** the memory is configured to store the determined blood pressure of at least one first heartbeat (11);
e) wherein the controller (7) is arranged to apply a varying cuff pressure (P_{cuff}) during at least one second heartbeat (12), and to receive the signal (PLET) during the at least one second heartbeat (12); and
f) wherein the controller (7) is arranged to determine a pressure volume relation based on the stored blood pressure of the at least one first heartbeat (11), the signal (PLET) of the at least one second heartbeat (12) and the varying cuff pressure (P_{cuff}) applied during the at least one second heartbeat (12), and to determine a value of the plethysmograph signal (PLET) corresponding to an unstretched arterial volume based on the pressure-volume relation, and set the determined value as a new set point (SET).

## Patentansprüche

1. Verfahren zum Korrigieren des Manschettendrucks bei einer nichtinvasiven kontinuierlichen Blutdruckmessung mit einem Plethysmographen in einer mit Fluid gefüllten Druckmanschette (2), die um ein Körperteil gewickelt ist, wobei das Verfahren die folgenden Schritte umfasst:
a) Variieren von Drücken (P_{Manschette}) innerhalb der Druckmanschette (2) und Messen der entsprechenden Signale des Plethysmographen;
b) Bestimmen eines Wertes des Plethysmographensignals (PLET) entsprechend einem unbelasteten ("unloaded") arteriellen Volumen; und Festlegen des bestimmten Wertes als anfänglichen Sollwertpunkt (SET);
c) Vergleichen von gemessenen Plethysmographensignalen (PLET) mit dem Sollwertpunkt;
d) Anpassen des Manschettendrucks (P_{Manschette}) in der Druckmanschette (2), um die Differenz zwischen den gemessenen Signalen (PLET) und dem Sollwertpunkt (SET) zu minimieren;
e) Überwachen des Manschettendrucks für eine Anzahl von Herzschlägen;
**dadurch gekennzeichnet, dass** das Verfahren des Weiteren die Schritte umfasst:
f) Speichern der Druckanpassungen der Manschette während mindestens eines ersten Herzschlags (11);
g) Anwenden eines variierenden Manschettendrucks (P_{Manschette}) auf die Druckmanschette (2) während mindestens eines zweiten Herzschlags (12) und Messen der entsprechenden Signale (PLET) des Plethysmographen;
h) Verwenden der gespeicherten Druckanpassungen des mindestens einen ersten Herzschlags (11) und des Manschettendrucks (P_{Manschette}) und des Plethysmographensignals (PLET) des mindestens einen zweiten Herzschlags (12), um eine Druck-Volumen-Beziehung zu bestimmen;
i) Bestimmen eines Wertes des Plethysmographensignals (PLET) entsprechend einem ungestressten arteriellen Volumen, basierend auf der bestimmten Beziehung; und Festlegen des bestimmten Wertes als neuen Sollwertpunkt (SET).

2. Verfahren nach Anspruch 1, umfassend Schritt j) der sich wiederholenden Schritte c) bis i).

3. Verfahren nach Anspruch 1, wobei der in Schritt g) angewendete variierende Manschettendruck (P_{Manschette}) einen Druck unterhalb des venösen Drucks in dem Körperteil distal zu der Manschette (2) umfasst.

4. Verfahren nach Anspruch 3, wobei der Druck unterhalb des venösen Drucks in dem Körperteil distal zu der Manschette (2) Werte unter dem diastolischen Blutdruck erreicht, vorzugsweise zwischen 0 und 50 mmHg, insbesondere ungefähr 30 mmHg.

5. Verfahren nach Anspruch 1, wobei der in Schritt g) angewendete variierende Manschettendruck (P_{Manschette}) eine dynamische Druckwellenform ist, in welcher der niedrigste Druck unterhalb des diastolischen Drucks in dem Körperteil liegt, was venösen Rückfluss in das Körperteil zulässt, und der höchste Druck oberhalb des systolischen Drucks in dem Körperteil liegt.

6. Verfahren nach Anspruch 1, wobei der in Schritt g) angewendete variierende Manschettendruck (P_{Manschette}) eine Rampenwellenform ist, in welcher der niedrigste Druck unterhalb des diastolischen Drucks in dem Körperteil liegt, was venösen Rückfluss in das Körperteil zulässt, und der höchste Druck oberhalb des systolischen Drucks in dem Körperteil liegt.

7. Verfahren nach Anspruch 1, wobei der in Schritt g) angewendete variierende Manschettendruck (P_{Manschette}) einen Übergang von einem niedrigen Druck unterhalb des diastolischen Drucks zu einem hohen Druck oberhalb des systolischen Drucks und danach mehrere Übergänge zwischen dem niedrigen und dem hohen Druck umfasst.

8. Verfahren nach Anspruch 1, und des Weiteren umfassend einen Schritt des Anwendens eines Manschettendrucks (P_{Manschette}) mit einer Schrittfunktion um ein vorbestimmtes Manschettendruckniveau herum, wobei das Niveau eines von (i) mittlerem arteriellem Druck und (ii) einem Druck auf halbem Wege zwischen systolischem und diastolischem Druck ist.

9. Verfahren nach Anspruch 1, wobei die Druck-Volumen-Beziehung durch das Signal des Plethysmographen (PLET) und den transmuralen Druck (Ptr) zwischen dem Blutgefäß des Körperteils und dem Manschettendruck (P_{Man-schette}) bestimmt wird.

10. Verfahren nach einem der vorhergehenden Ansprüche, wobei der während Schritt g) angewendete variierende Manschettendruck (P_{Manschette}) so gewählt wird, dass der transmurale Druck (Ptr) zwischen dem Blutgefäß des Körperteils und dem Manschettendruck (P_{Manschette}) über einen vorbestimmten Bereich festgelegt wird.

11. Verfahren nach einem der vorhergehenden Ansprüche, wobei der während Schritt g) angewendete variierende Manschettendruck (P_{Manschette}) basierend auf dem gespeicherten Blutdruck des mindestens einen ersten Herzschlags (11) bestimmt wird.

12. Verfahren nach einem der vorhergehenden Ansprüche, wobei in Schritt b) der Wert des Sollwertpunkts das Volumen ist, bei dem die Amplitude des Plethysmographensignals maximal ist.

13. Verfahren nach einem der vorhergehenden Ansprüche, umfassend den Schritt des Bestimmens des zentralen Blutdrucks basierend auf dem gemessenen Blutdruck innerhalb des Körperteils, vorzugsweise des Fingers.

14. Verfahren nach einem der vorhergehenden Ansprüche, wobei in Schritt i) der neue Sollwertpunkt in Beziehung zu dem Volumen bestimmt wird, wobei eine Compliance des gemessenen Körperteils, wie der Fingerarterie, maximal ist.

15. Vorrichtung (1) zum Korrigieren des Manschettendrucks bei einer nichtinvasiven Blutdruckmessung mit einem Plethysmographen in einer mit Fluid gefüllten Druckmanschette (2), die um ein Körperteil, wie einen Finger, gewickelt ist, umfassend:
a) eine Druckmanschette (2), die ausgestattet ist mit:
a. einer Blase, um um das Körperteil gewickelt zu werden und einen Gegendruck auszuüben;
b. einer Lichtquelle, um Licht durch das Körperteil zu senden; und
c. einem Lichtdetektor, um das Licht zu detektieren, welches durch das Körperteil hindurch gelangt, und um in Abhängigkeit von der Menge des detektierten Lichts ein Signal (PLET) bereitzustellen;
b) einen Druckgenerator (9), der ausgestaltet ist, um der Blase druckbeaufschlagtes Fluid zuzuführen;
c) eine Steuerung (7), die ausgestaltet ist, um den Druck des der Blase zugeführten Fluids basierend auf dem Signal (PLET) anzupassen, um Drücke (P_{Manschette}) innerhalb der Druckmanschette (2) zu variieren, um die entsprechenden Signale (PLET) zu messen, um einen Wert des Plethysmographensignals (PLET) zu bestimmen, der einem unbelasteten arteriellen Volumen entspricht, um einen anfänglichen Sollwertpunkt (SET) festzulegen, um gemessene Plethysmographensignale (PLET) mit dem Sollwertpunkt (SET) zu vergleichen, um den Druck anzupassen, um die Differenz zwischen den gemessenen Signalen (PLET) und dem Sollwertpunkt (SET) zu minimieren, um den Manschettendruck für eine Anzahl von Herzschlägen zu überwachen, und um den Blutdruck innerhalb des Körperteils zu bestimmen; und
d) einen Speicher,
**dadurch gekennzeichnet, dass** der Speicher ausgestaltet ist, um den bestimmten Blutdruck von mindestens einem ersten Herzschlag (11) zu speichern;
e) wobei die Steuerung (7) angeordnet ist, um einen variierenden Manschettendruck (P_{Manschette}) während mindestens eines zweiten Herzschlags (12) anzuwenden und das Signal (PLET) während des mindestens einen zweiten Herzschlags (12) zu empfangen; und
f) wobei die Steuerung (7) angeordnet ist, um eine Druck-Volumen-Beziehung basierend auf dem gespeicherten Blutdruck des mindestens einen ersten Herzschlags (11), dem Signal (PLET) von dem mindestens einen zweiten Herzschlag (12) und dem variierenden Manschettendruck (P_{Manschette}) zu bestimmen, der während des mindestens einen zweiten Herzschlags (12) angewendet wurde, und um einen Wert des Plethysmographensignals (PLET), der einem ungestressten arteriellen Volumen entspricht, basierend auf der Druck-Volumen-Beziehung zu bestimmen und den bestimmten Wert als neuen Sollwertpunkt (SET) festzulegen.

## Revendications

1. Procédé de correction de pression de brassard dans une mesure continue non invasive de tension artérielle à l'aide d'un pléthysmographe dans un brassard de pression (2) rempli de fluide enroulé autour d'une partie corporelle, le procédé comprenant les étapes de :
a) variation des pressions (P_{brassard}) à l'intérieur du brassard de pression (2) et mesure des signaux correspondants du pléthysmographe ;
b) détermination d'une valeur du signal de pléthysmographe (PLET) correspondant à un volume artériel non chargé ; et réglage de la valeur déterminée en tant que point de consigne (SET) initial ;
c) comparaison des signaux de pléthysmographe (PLET) mesurés au point de consigne ;
d) ajustement de la pression de brassard (P_{brassard}) dans le brassard de pression (2) afin de réduire au minimum la différence entre les signaux mesurés (PLET) et le point de consigne (SET) ;
e) surveillance de la pression de brassard pendant un certain nombre de battements cardiaques ;
**caractérisé en ce que** le procédé comprend en outre les étapes de :
f) enregistrement des ajustements de pression du brassard pendant au moins un premier battement cardiaque (11) ;
g) application d'une pression de brassard (P_{brassard}) variable au brassard de pression (2) pendant au moins un second battement cardiaque (12) et mesure des signaux (PLET) correspondants du pléthysmographe ;
h) utilisation des ajustements de pression enregistrés dudit au moins un premier battement cardiaque (11) et de la pression de brassard (P_{brassard}) et du signal de pléthysmographe (PLET) dudit au moins un second battement cardiaque (12) afin de déterminer une relation pression-volume ;
i) détermination d'une valeur du signal de pléthysmographe (PLET) correspondant à un volume artériel non étiré, sur la base de la relation déterminée ; et réglage de la valeur déterminée en tant que nouveau point de consigne (SET).

2. Procédé selon la revendication 1, comprenant l'étape j) de répétition des étapes c) à i).

3. Procédé selon la revendication 1, dans lequel la pression de brassard (P_{brassard}) variable appliquée à l'étape g) comprend une pression inférieure à la tension veineuse dans la partie corporelle distale par rapport au brassard (2).

4. Procédé selon la revendication 3, dans lequel la pression inférieure à la tension veineuse dans la partie corporelle distale par rapport au brassard (2) atteint des valeurs inférieures à la tension artérielle diastolique, de préférence entre 0 et 50 mmHg, en particulier autour de 30 mmHg.

5. Procédé selon la revendication 1, dans lequel la pression de brassard (P_{brassard}) variable appliquée à l'étape g) est une forme d'onde de pression dynamique, dans laquelle la pression la plus basse est inférieure à la tension diastolique dans la partie corporelle, ce qui permet un retour veineux dans la partie corporelle, et la pression la plus élevée est supérieure à la tension systolique dans la partie corporelle.

6. Procédé selon la revendication 1, dans lequel la pression de brassard (P_{brassard}) variable appliquée à l'étape g) est une forme d'onde en rampe, dans laquelle la pression la plus basse est inférieure à la tension diastolique dans la partie corporelle, ce qui permet un retour veineux dans la partie corporelle, et la pression la plus élevée est supérieure à la tension systolique dans la partie corporelle.

7. Procédé selon la revendication 1, dans lequel la pression de brassard (P_{brassard}) variable appliquée à l'étape g) comprend une transition depuis une basse pression inférieure à la tension diastolique vers une pression élevée supérieure à la tension systolique, puis de multiples transitions entre lesdites pressions basse et élevée.

8. Procédé selon la revendication 1 et comprenant en outre une étape d'application d'une pression de brassard (P_{brassard}) présentant une fonction en escalier autour d'un niveau de pression de brassard prédéterminé, lequel niveau est une pression parmi (i) la tension artérielle moyenne et (ii) une pression à mi-chemin entre la tension systolique et diastolique.

9. Procédé selon la revendication 1, dans lequel la relation pression-volume est déterminée par le signal du pléthysmographe (PLET) et la pression transmurale (Ptr) entre le vaisseau sanguin de la partie corporelle et la pression de brassard (P_{brassard}).

10. Procédé selon l'une quelconque des revendications précédentes, dans lequel la pression de brassard (P_{brassard}) variable appliquée pendant l'étape g) est choisie de telle sorte que la pression transmurale (Ptr) entre le vaisseau sanguin de la partie corporelle et la pression de brassard (P_{brassard}) est réglée sur une plage prédéterminée.

11. Procédé selon l'une quelconque des revendications précédentes, dans lequel la pression de brassard (P_{brassard}) variable appliquée pendant l'étape g) est déterminée sur la base de la tension artérielle enregistrée dudit au moins un premier battement cardiaque (11).

12. Procédé selon l'une quelconque des revendications précédentes, dans lequel, à l'étape b), la valeur du point de consigne est le volume dans lequel l'amplitude du signal de pléthysmographe est maximale.

13. Procédé selon l'une quelconque des revendications précédentes, comprenant l'étape de détermination de la tension artérielle centrale sur la base de la tension artérielle mesurée à l'intérieur de la partie corporelle, de préférence le doigt.

14. Procédé selon l'une quelconque des revendications précédentes, dans lequel, à l'étape i), le nouveau point de consigne est déterminé par rapport au volume où une conformité de la partie corporelle mesurée, telle que l'artère du doigt, est maximale.

15. Dispositif (1) de correction de la pression de brassard dans une mesure non invasive de tension artérielle à l'aide d'un pléthysmographe dans un brassard de pression (2) rempli de fluide enroulé autour d'une partie corporelle, telle qu'un doigt, comprenant :
a) un brassard de pression (2), muni de :
a. une poche, destinée à s'enrouler autour de la partie corporelle et à appliquer une contre-pression ;
b. une source de lumière, destinée à envoyer de la lumière à travers la partie corporelle ; et
c. un détecteur de lumière, destiné à détecter la lumière passée à travers la partie corporelle et à fournir un signal (PLET) en fonction de la quantité de lumière détectée ;
b) un générateur de pression (9) configuré pour fournir du fluide sous pression à la poche ;
c) un dispositif de commande (7) configuré pour ajuster la pression du fluide fourni à la poche sur la base du signal (PLET), pour faire varier des pressions (P_{brassard}) à l'intérieur du brassard de pression (2), pour mesurer les signaux (PLET) correspondants, pour déterminer une valeur du signal de pléthysmographe (PLET) correspondant à un volume artériel non chargé afin de régler un point de consigne (SET) initial, pour comparer des signaux de pléthysmographe (PLET) mesurés au point de consigne (SET), pour ajuster la pression afin de réduire au minimum la différence entre les signaux mesurés (PLET) et le point de consigne (SET), pour surveiller la pression de brassard pendant un certain nombre de battements cardiaques et pour déterminer la tension artérielle à l'intérieur de la partie corporelle ; et
d) une mémoire,
**caractérisé en ce que** la mémoire est configurée pour enregistrer la tension artérielle déterminée d'au moins un premier battement cardiaque (11) ;
e) le dispositif de commande (7) étant agencé pour appliquer une pression de brassard (P_{brassard}) variable pendant au moins un second battement cardiaque (12) et pour recevoir le signal (PLET) pendant ledit au moins un second battement cardiaque (12) ; et
f) le dispositif de commande (7) étant agencé pour déterminer une relation pression-volume sur la base de la tension artérielle enregistrée dudit au moins un premier battement cardiaque (11), du signal (PLET) dudit au moins un second battement cardiaque (12) et de la pression de brassard (P_{brassard}) variable appliquée pendant ledit au moins un second battement cardiaque (12) et pour déterminer une valeur du signal de pléthysmographe (PLET) correspondant à un volume artériel non étiré sur la base de la relation pression-volume et pour régler la valeur déterminée en tant que nouveau point de consigne (SET).
